# EUROPEAN PATENT SPECIFICATION

(11) **EP 4 084 792 B1**
(45) Date of publication and mention of the grant of the patent: **02.09.2026**
(21) Application number: 20909105.7
(22) Date of filing: 29.12.2020
(51) Int. Cl.: A61K 31/43, A61P 3/10, A61P 31/04, A61P 35/00, C07D 499/70

(54) **PENICILLIN DERIVATIVES AND METHOD FOR THEIR SYNTHESIS**
PENICILLINDERIVATE UND VERFAHREN ZU IHRER SYNTHESE
DÉRIVÉS DE PÉNICILLINE ET LEUR MÉTHODE DE SYNTHÈSE

(30) Priority: 31.12.2019 TR 201922977
(43) Date of publication of application: 09.11.2022
(73) Proprietor: T.C. Erciyes Üniversitesi, 38280 Talas/Kayseri (TR)
(72) Inventor: SARIPINAR, Emin, Kayseri (TR); ILHAN, Ilhan Oezer, Kayseri (TR); ÇADIR, Mehmet, Kayseri (TR)
(74) Representative: Yamankaradeniz, Kemal
(86) International application number: PCT/TR2020/051443
(87) International publication number: WO 2021/137835

(56) References cited:
- EP-A1- 0 084 948
- DD-A1- 25 568
- JP-A- S5 772 985
- US-A- 3 923 788
- US-A- 4 342 772

## Description

### Technical Field

The present invention relates to new penicillin derivatives as defined in the claims having the potential of being effectively used against microbes and bacteria which have attained resistance to perform many useful pharmaceutical activities such as antiprotozoal, antibacterial, antimalarial, anticoagulant, antiviral, anticancer and antihypertensive, anticancerogenic, antifungal, trypanocidal, antisecretory, antidiarrheatic, antileukemic, cardiotonic, anticancer, and methods of preparation thereof.

### Prior Art

Penicillin (Ampicillin) is one of the oldest and most commonly used antibiotics. It is the first medicine which has been used against bacterial infections. Many drugs that belongs to the class of penicillin. Penicillin is the most preferred antibiotics owing to their powerful action against infections, being cheap and having lesser toxic effects. Alexander Fleming discovered in the year 1928 in London that some bacteria species cannot reproduce around *Penicillium notatum* species of mold fungi. Fleming who discovered the antibacterial this fungus has pioneered the development of penicillin which is one of the most useful drugs of the history of medicine.

Penicillin is being used effectively in many diseases such as peritonsillar abscess, bacterial pneumonia, lung abscess, a portion of bladder and kidney inflammations, prostatic inflammation, inflammatory skin burns, pharyngitis, intraocular inflammation, bone inflammations, middle ear inflammations, mammary inflammation, brain abscess, meningitis, blood poisonings (septicemias), laryngitis. The broad range of diseases in which penicillin are used therapeutically and the usage of them without paying attention to their dosages has developed resistance to this medicine in various microorganisms. Currently, the same disease can be treated by administering much higher doses of penicillin as compared to the past.

In recent years, studies are being performed to break the resistance of beta-lactamase enzyme producing bacteria against penicillin. In one of the developed methods, penicillin and sulbactam substance in the ratio of half thereof is combined. And in another technique, penicillin and potassium clavulanate which is the clavulanic acid powder is being applied by combining in a ratio of quarter thereof.

**In** patent document number TR2013 / 02167 related to the subject which is present in the prior art, antibacterial formulations developed to be used in the symptomatic and/or prophylactic and/or therapeutic treatment of infections of the upper respiratory tract or chronic middle ear inflammation originating from *Streptococcus pneumoniae, Haemophilus influenzae* and *Moraxella catarrhalis;* tonsillo - pharyngitis and sinusitis caused by *Streptococcus pneumoniae, Haemophilus influenzae, Moraxella catarrhalis* and *Streptococcus pyogenes;* lower respiratory tract infections such as lobar pneumonia and bronchopneumonia caused by *Steptococcus pneumoniae, Haemophilus influenzae and Morexella catarrhalis;* skin and soft tissue infections caused by *Staphylococcus aureus* and *Streptococcus pyogenes* are disclosed. The said formulations include the combination of a penicillin class beta - lactam antibiotic and/or its pharmaceutically acceptable derivatives with a penicillin class beta - lactamase inhibitor and / or its pharmaceutically acceptable derivatives.

**In** the patent document number US2014088069A1 which is another document present in the prior art, an antibacterial composition formed by the combination of a beta-lactam antibiotic having binding affinity for bacterial penicillin binding protein 2 and thienopyridine or a non - antibiotic compound which is not thienopyridine, and its use for therapeutic purposes is disclosed.

Another patent document numbered DD25568A related to process for the preparation of antibiotically active addition compounds of penicillin. **In** said document for composition [(HX)p (MeY)n]; Hx represents a penicillin or the radical of another organic acid, salt or ester; Me represents preferably alkali metal series; Y represents a penicillin or another organic acid or mineral, or a salt or ester thereof. D1 document mentions use of salt like barbituric acid and compound obtained by using barbituric acid and its binding to 6-APA, however there is no information on the synthesis of pyrimidine derivatives and their conversion to acid chloride and their reaction with 6-aminopenicillinic acid (6-APA).

When the studies present in the prior art are reviewed, studies performed aimed at breaking the resistance formed against penicillin are based in general terms on combining different medicines.

In this respect, it is seen that a need is present for new penicillin derivatives which are suitable for use as a standalone antibacterial medicine, which increases patient's compliance, whose efficacy on resistant bacteria as compared to penicillin has been enhanced, and novel methods aimed at the production of the same.

### Brief description of the invention

The present invention relates to penicillin derivatives which meet the above-mentioned requirements, which eliminate all disadvantages and which offer some additional advantages, and a method for synthesizing the same.

The prioritized objective of the invention is to obtain imidazole (parabanic acid) and pyrimidine based new penicillin derivatives which are not present in the literature.

Another objective of the invention is to obtain penicillin derivatives suitable to be used in the treatment of medical diseases such as cancer, bacterial diseases and diabetes.

Another objective of the invention is to develop methods related to synthesizing of new penicillin derivatives which facilitate the treatment by breaking the resistance of the bacteria against penicillin.

Another objective of the invention is to obtain imidazole (parabanic acid) and pyrimidine based new acetic acid derivatives which are not present in the literature.

In order to fulfil the above-mentioned objectives, the present invention is a method for synthesizing penicillin derivatives having chemical formulae (I) comprising the steps of; 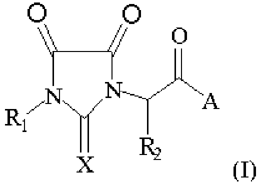 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining parabanic acid ester derivative from the reaction of amino acid-based urea derivative with oxalyl chloride
iii. conversion of parabanic acid ester derivative to parabanic acid derivative by hydrolysis
iv. conversion of parabanic acid derivative to parabanic acetyl chloride by chlorination
v. obtaining a parabanic acid-based penicillin derivative from the reaction of parabanic acetyl chloride with the 6-aminopenicillinic acid (6-APA) compound.

In order to fulfil the above-mentioned objectives, the present invention is a method for synthesizing penicillin derivatives having chemical formulae (II) comprising the steps of; 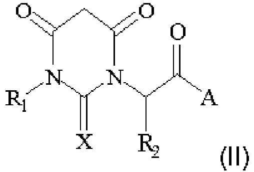 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining pyrimidine based acetic acid ester derivative from the reaction of amino acid-based urea derivative with malonyl chloride
iii. conversion of pyrimidine based acetic acid ester derivative to pyrimidine based acetic acid derivative by hydrolysis
iv. conversion of pyrimidine based acetic acid derivative to pyrimidine-based acetyl chloride by chlorination
v. obtaining a pyrimidine-based penicillin derivative from the reaction of pyrimidine-based acetyl chloride with the 6-aminopenicillinic acid (6-APA) compound.

Structural and characteristic features of the invention and all of its advantages will be understood more clearly utilizing detailed description. Therefore, it should be appreciated by considering the detailed description.

### Detailed Description of the Invention

In this detailed description, methods for the synthesis of penicillin derivatives are disclosed aimed at a better understanding of the subject and without forming any limiting effects.

A method for the synthesis of penicillin derivatives having chemical formula (I) comprising the steps of; 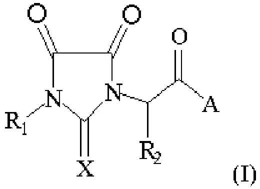 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining parabanic acid ester derivative from the reaction of amino acid-based urea with oxalyl chloride
iii. conversion of parabanic acid ester derivative to parabanic acid derivative by hydrolysis
iv. conversion of parabanic acid derivative to parabanic acetyl chloride by chlorination
v. obtaining a parabanic based penicillin derivative from the reaction of parabanic acetyl chloride with the 6-aminopenicillinic acid (6-APA) compound.

In an embodiment of the invention, in process step number (i) phenylglycine methyl ester compound is reacted with phenyl thioisocyanate, and phenylglycine based urea derivative, more specifically 2-phenyl-2-(3-phenylthiourea) acetic acid ester is obtained. The respective reaction is as follows.

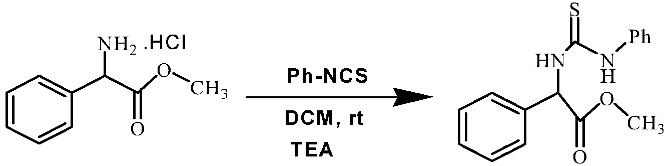

**In** an embodiment of the method of the invention, in the reaction in which said S element has been replaced with the O element, phenylglycine based urea derivative is synthesized.

According to an embodiment of the method of invention, 2-phenyl-2-(3-phenylthiourea acetic acid ester which has been synthesized in process step number (i) is reacted with oxalyl chloride in process step number (ii) and 2-(4,5-dioxo-3-phenyl-2-thiooxoimidazolidine-1-yl)-2-(phenyl) acetic acid methyl ester is obtained as an intermediate product. **In** an embodiment of the invention, 2-(4,5-dioxo-3-phenyl-2-thiooxoimidazolidine-1-yl)-2-(phenyl) acetic acid methyl ester synthesized in process step number (ii) is converted to 2-(4,5-dioxo-3-phenyl-2-thiooxoimidazolidine-1-yl)-2-(phenyl) acetic acid derivative in process step number (iii) by hydrolysis. The respective reaction is as follows.

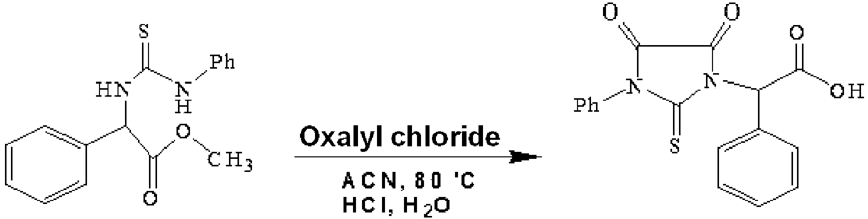

In an embodiment of the method of the invention, in the reaction in which said S element has been replaced with the O element, parabanic acid ester derivative is synthesized.

In an embodiment of the invention, 2-(4,5-dioxo-3-phenyl- 2-thiooxoimidazolidine-1-yl)-2-(phenyl) acetic acid obtained in process step number (iii) is chlorinated in process step number (iv) with a component selected from the group consisting of thionyl chloride, phosphorus pentachloride, and phosphorus trichloride. According to an embodiment, 2-(4,5-dioxo-3-phenyl-2-thiooxoimidazolidine-1-yl)-2-(phenyl) acetyl chloride is obtained preferably by chlorinating 2-(4,5-dioxo-3-phenyl-2-thiooxoimidazolidine-1-yl)-2-(phenyl) acetic acid. The respective reaction is as follows.

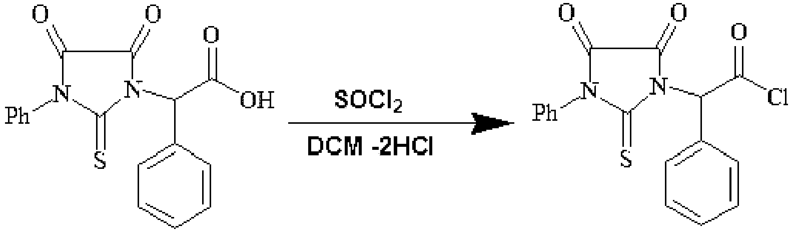

**In** an embodiment of the method of the invention, in the reaction in which said S element has been replaced with the O element, parabanic acetyl chloride derivative is synthesized.

**In** an embodiment of the invention, 2-(4,5-dioxo-3-phenyl-2-thiooxoimidazolidine-1-yl)-2-(phenyl) acetyl chloride synthesized in process step number (iv) is reacted with 6-aminopenicillinic acid (6-APA) compound or esters and amides thereof in process step number (v), and sodium 6-(2-(4,5-dioxo-3-phenyl-2-thiooxoimidazolidine-1-yl)-2-phenylacetamido)-3,3-dimethyl-7-oxo-4-thya-1-aza-bicycle [3.2.0] heptane-2-carboxilate synthesis is attained. The synthesized said component is a new parabanic acid-based penicillin derivative which is not present in the literature. The respective reaction is as follows.

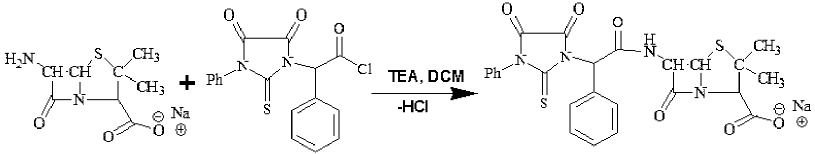

In an embodiment of the method of the invention, in the reaction in which said S element has been replaced with the O element, parabanic acid-based penicillin derivative is synthesized.

A method for the synthesis of penicillin derivatives having chemical formula (II) comprising the steps of; 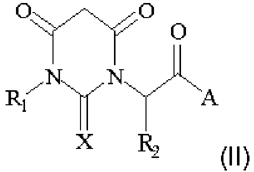 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining pyrimidine based acetic acid ester derivative from the reaction of amino acid-based urea derivative with malonyl chloride
iii. conversion of pyrimidine based acetic acid ester derivative to pyrimidine based acetic acid derivative by hydrolysis
iv. conversion of pyrimidine based acetic acid ester derivative to pyrimidine based acetyl chloride by chlorination
v. obtaining a pyrimidine based penicillin derivative from the reaction of pyrimidine based acetyl chloride with the 6-aminopenicillinic acid (6-APA) compound .

In an embodiment of the invention, phenylglycine methyl ester compound is reacted with phenyl thioisocyanate in process step number (i), and phenylglycine based urea derivative, more specifically 2-phenyl-2-(3-phenylthiourea) acetic acid ester is obtained. The respective reaction is as follows.

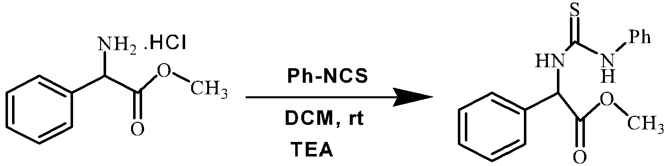

In an embodiment of the method of the invention, in the reaction in which said S element has been replaced with the O element, phenylglycine based urea derivative is synthesized.

According to an embodiment of the method of invention, phenylglycine based urea derivative synthesized in process step number (i) is reacted with malonyl chloride in process step number (ii), and 2-(4,6-dioxo-3-phenyl-2-thioxo-tetrahydropyridine-1 (2H)-yl)-2-phenylacetic acid methyl ester is obtained. In an embodiment of the invention, 2-(4,6-dioxo-3-phenyl-2-thioxo-tetrahydropyridine-1(2H)-yl)-2-phenylacetic acid methyl ester synthesized in process step number (ii) is converted to 2-(4, 6-dioxo-3-phenyl-2 -thioxo-tetrahydropyridine-1-(2H)-yl)-2-phenylacetic acid derivative in process step number (iii) by hydrolysis. The respective reaction is as follows.

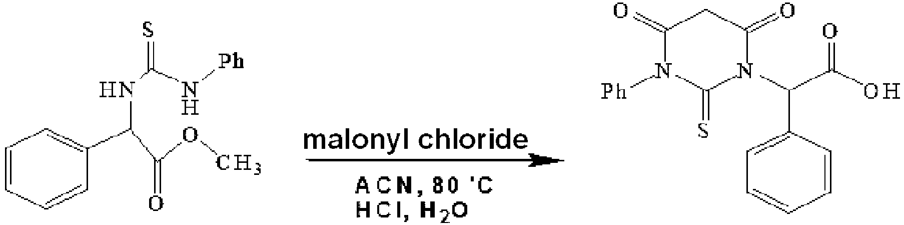

In an embodiment of the method of the invention, in the reaction in which said S element has been replaced with the O element, pyrimidine based acetic acid ester derivative is synthetized.

In an embodiment of the invention, 2-(4,6-dioxo-3-phenyl-2-thioxo-tetrahydropyridine-1-(2H)-yl)-2-phenylacetic acid obtained in process step number (iii) is chlorinated in process step number (iv) with a component selected from the group consisting of thyonyl chloride, phosphorus pentachloride, and phosphorus trichloride. According to an embodiment, 2-(4,6-dioxo-3-phenyl-2-thioxo-tetrahydropyridine-1-(2H)-yl)-2-phenylacetyl chloride is obtained preferably by chlorinating 2-(4,6-dioxo-3-phenyl-2-thioxo-tetrahydropyridine-1-(2H)-yl)-2-phenylacetic acid. The respective reaction is as follows.

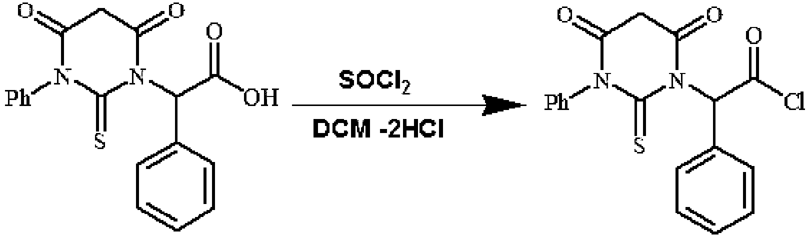

**In** an embodiment of the method of the invention, in the reaction in which said S element has been replaced with the O element, pyrimidine-based acetyl chloride derivative is synthesized.

**In** an embodiment of the invention, 2-(4,6-dioxo-3-phenyl-2-thioxo-tetrahydropyridine-1-(2H)-yl)-2-phenylacetyl chloride synthesized in process step number (iv) is reacted with 6-aminopenicillinic acid (6-APA) compound in process step number (v), and sodium 6-(2-(4,6-dioxo-3-phenyl-2-thioxo-tetrahydropyridine-1-(2H)-yl)-2-phenylacetamido)-3,3-dimethyl-7-oxo-4-thya-1-aza-bicycle[3.2.0] heptane-2- carboxylate synthesis is attained. The synthesized said component is a new penicillin derivative which is not present in the literature. The respective reaction is as follows.

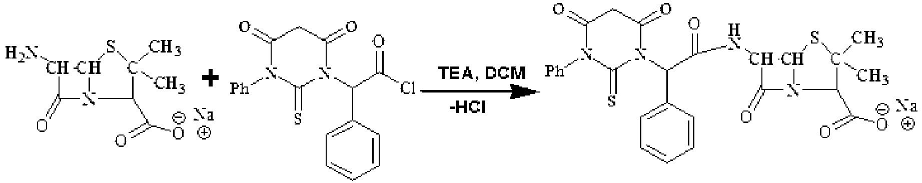

In an embodiment of the method of the invention, in the reaction in which said S element has been replaced with the O element, pyrimidine-based penicillin derivative is synthesized.

Penicillin derivatives of the invention are suitable for use as a therapeutic medicine for medicinal diseases such as cancer, bacterial diseases and diabetes. Also, instead of 6-APA all penicillin derivatives can be synthesized for these studies too.

It is conceived at the same time that these compounds will have many useful antiprotozoal pharmacological activities such as antibacterial, antimalarial, antidiuretic, anticoagulant, antiviral, anticancer and antihypertensive, anticancerogenic, antifungal, trypanocidal, antisecretory, antileukemic,, cardiotonic, anticancer, and anticoagulant.

Structural characterization of the penicillin derivatives synthesized with the method of the invention has been done by FT-IR, ¹H-NMR, ¹³C-NMR, COSY-NMR and HETCOR-NMR techniques.

For this purpose, phenylglycine methyl ester compound weighed in the stoichiometric ratios has been dissolved with dichloromethane (DCM). Equivalent molar amounts of phenylizothiocyanate and triethylamine (TEA) have been taken and added into the solution and the reaction has been mixed at room temperature (RT) for a period of 24 hours. At the end of the reaction, the solvent is removed from the rotavapor (rotary evaporator) and the oily portion is obtained. The oily portion is extracted in the presence of aqueous NaHCO₃ and ethyl acetate and the organic portion is taken and the sample has been obtained by removing ethyl acetate. Its characterization has been performed by various spectroscopic techniques after the drying process has been done.

A series of trials have been done to determine the conditions of the reaction of the obtained phenyl-2-(3-phenylthiourea) acetic acid ester compound with oxalyl chloride or malonyl chloride. As a result of this, reaction conditions have been determined as the following.

After phenyl-2-(3-phenylthiourea) acetic acid ester compound weighed in the stoichiometric ratios has been dissolved with acetonitrile, equivalent molar amounts of oxalyl chloride or malonyl chloride have been added over it and have been left at reflux for 6 hours. While the reaction is going on, equivalent molar amounts of the hydrochloric acid solution has been added at the end of the 6^{th} hour and the reaction has been completed. The oily portion has been crystallized from cyclohexane by removing the solvent from the rotavapor. Its characterization has been performed by various spectroscopic techniques after drying process has been done.

In another study, by adding thyonyl chloride over the acetic acid compound containing imidazole and pyrimidine ring weighed in the stoichiometric ratios the reaction has been continued for 48 hours in a water bath in stoichiometric ratio. Its characterization has been performed by various spectroscopic techniques at the end of the reaction after the drying process of the product is performed by crystallizing from xylene.

A series of trials have been done to determine the conditions of the reaction of 6-APA compound; and as a result of this, reaction conditions have been determined as the following.

By weighing with acetic acid chloride derivatives in equivalent molar amounts containing (6-APA) compound and imidazole or pyrimidine ring weighed in the stoichiometric ratios, a few drops of TEA have been added over it. These have been placed inside a reaction flask and 30 ml of DCM have been added and stirred for 36 hours at room temperature under reflux. After removal of the solvent, the oily portion has been washed in ethyl acetate and diethyl ether and passed on to characterization by various techniques after the drying process has been completed.

At the end of all these characterization studies, it has been determined that penicillin derivatives of the invention having biologically active imidazole and pyrimidine rings are in the antibiotic structure which is suitable for use against bacterial infections.

## Claims

1. A method for the synthesis of penicillin derivatives having chemical formula (I) **characterized by comprising** the process steps of; 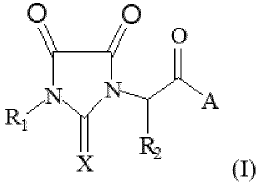 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining parabanic acid ester derivative from the reaction of amino acid-based urea with oxalyl chloride
iii. conversion of parabanic acid ester derivative to parabanic acid derivative by hydrolysis
iv. conversion of parabanic acid derivative to parabanic acetyl chloride by chlorination
v. obtaining a parabanic based penicillin derivative from the reaction of parabanic acetyl chloride with the 6-aminopenicillinic acid (6-APA) compound.

2. The method according to claim 1, **characterized in that** in process step number (i) said amino acid-based urea derivative is 2-phenyl-2-(3-phenylthiourea) acetic acid ester and synthesized by the below reaction.

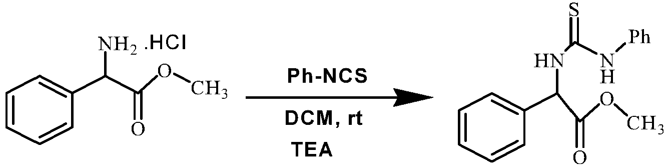

3. The method according to claim 2, **characterized in that** in process step number (ii) said parabanic acid ester derivative is 2-(4,5-dioxo-3-phenyl-2-thiooxoimidazolidine-1-yl)-2-(phenyl) acetic acid methyl ester and synthesized by the below reaction.

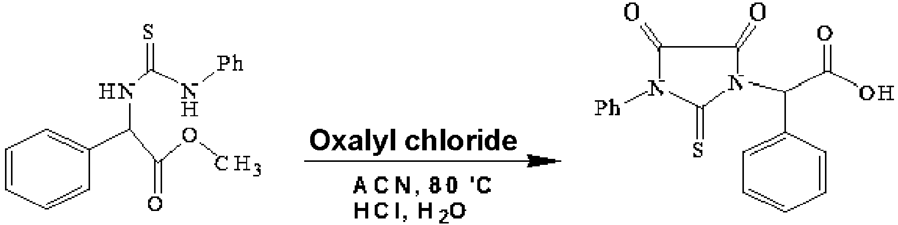

4. The method according to claim 3, **characterized in that** in process step number (iii) said parabanic acid derivative is 2-(4,5-dioxo-3-phenyl-2-thiooxoimidazolidine-1-yl)-2-(phenyl) acetic acid.

5. The method according to claim 4, **characterized in the** process step number (iv) said chlorinating process is performed with a component selected from the group consisting of thionyl chloride, phosphorus pentachloride, and phosphorus trichloride.

6. The method according to any one of claim 4 and claim 5, **characterized in the** process step number (iv) said parabanic acetyl chloride is 2-(4,5-dioxo-3-phenyl-2-thiooxoimidazolidine-1-yl)-2-(phenyl) acetyl chloride and synthesized by the below reaction.

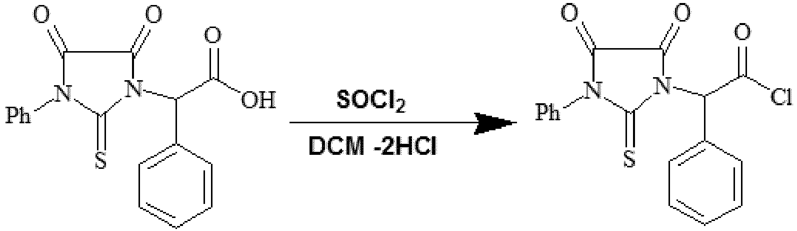

7. The method according to claim 6, **characterized in the** process step number (v) said parabanic acid-based penicillin derivative is sodium 6-(2-(4,5-dioxo-3-phenyl-2-thiooxoimidazolidine-1-yl)-2-phenylacetamido)-3,3-dimethyl-7-oxo-4-thya-1-aza-bicycle[3.2.0] heptane-2-carboxilate, and synthesized by the below reaction.

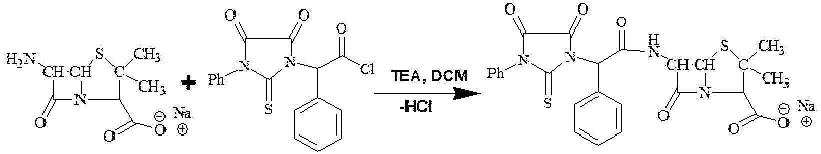

8. The method according to any one of the claims 2 to 7, **wherein** in the compounds said "S" element has been substituted with the "O" element.

9. A method for the synthesis of penicillin derivatives having chemical formula (II) **characterized by comprising** the process steps of; 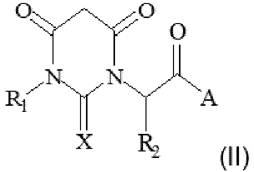 wherein R₁ and R₂ represent the 4,5,6,7-membered aromatic, heterocyclic or alkyl groups; A represents the 6-aminopenicillinic acid (6-APA) compound; X represents the O or S element
i. obtaining amino acid-based urea derivative from the reaction of amino acid with isocyanate or thioisocyanate
ii. obtaining pyrimidine based acetic acid ester derivative from the reaction of amino acid-based urea derivative with malonyl chloride
iii. conversion of pyrimidine based acetic acid ester derivative to pyrimidine based acetic acid derivative by hydrolysis
iv. conversion of pyrimidine based acetic acid ester derivative to pyrimidine-based acetyl chloride by chlorination
v. obtaining a pyrimidine-based penicillin derivative from the reaction of pyrimidine-based acetyl chloride with the 6 - aminopenicillinic acid (6 - APA) compound.

10. The method according to claim 9, **characterized in that** in process step number (i) said amino acid-based urea derivative is 2-phenyl-2-(3-phenylthiourea) acetic acid ester and synthesized by the below reaction.

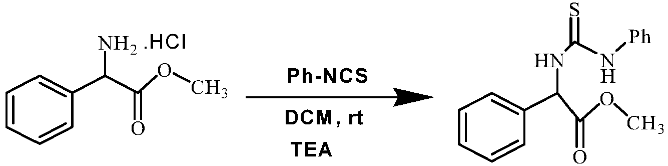

11. The method according to claim 10, **characterized in that** in process step number (ii) said pyrimidine based acetic acid ester derivative is 2-(4,6-dioxo-3-phenyl-2-thioxo-tetrahidropyrimidine-1-(2H)-yl)-2-phenylacetic acid methyl ester and synthesized by the below reaction.

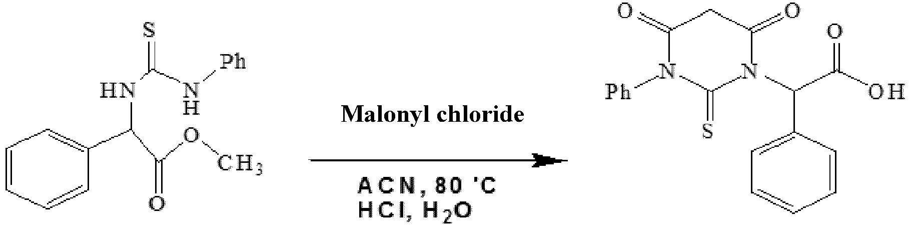

12. The method according to claim 11, **characterized in that** in process step number (iii) said pyrimidine based acetic acid derivative is 2-(4,6-dioxo-3-phenyl-2-thioxo-tetrahidropyrimidine-1-(2H)-yl)-2-phenylacetic acid.

13. The method according to claim 12, **characterized in that** in process step number (iv) said the chlorinating process is performed with a component selected from the group consisting of thionyl chloride, phosphorus pentachloride, and phosphorus trichloride.

14. The method according to any one of claim 12 to claim 13 **characterized in that** in process step number (iv) said pyrimidine-based acetyl chloride is 2-(4,6-dioxo-3-phenyl-2- thioxo-tetrahidropyrimidine-1-(2H)-yl)-2-phenylacetyl and synthesized by the below reaction.

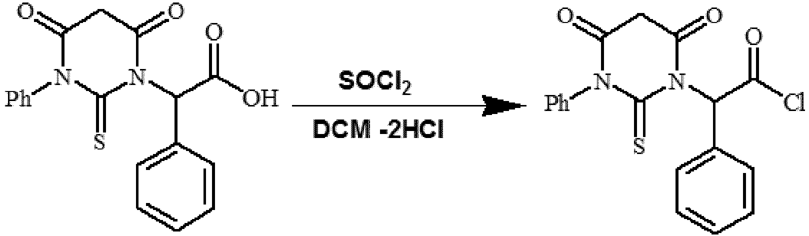

15. The method according to claim 14, **characterized in that** in process step number (v) said pyrimidine-based penicillin derivative is sodium 6-(2-(4,6-dioxo-3-phenyl-2-thioxo-tetrahidropyrimidine-1-(2H)-yl)-2-phenylacetamido)-3,3-dimethyl-7-oxo-4-thya-1-aza-bicycle[3.2.0]heptane-2-carboxilate and synthesized by the below reaction.

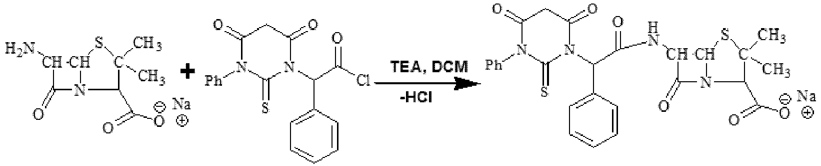

16. The method according to any one of claim 10 to claim 15, **wherein** in the compounds said "S" element has been substituted with the "O" element.

## Patentansprüche

1. Verfahren zur Synthese von Penicillinderivaten mit der chemischen Formel **(I), dadurch gekennzeichnet, dass** es die folgenden Vorgangsschritte umfasst; 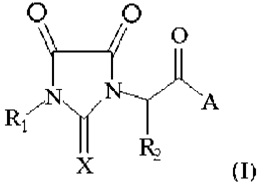 wobei R₁ und R₂ die 4,5,6,7-gliedrigen aromatischen, heterocyclischen oder Alkylgruppen darstellen; A die 6-Aminopenicillinsäure(6-APA)-Verbindung darstellt; X das O- oder S-Element darstellt
i. Erlangen eines Harnstoffderivats auf Aminosäurebasis aus der Reaktion von Aminosäure mit Isocyanat oder Thioisocyanat
ii. Erlangen des Parabansäureesterderivats aus der Reaktion von Harnstoff auf Aminosäurebasis mit Oxalylchlorid
iii. Umwandeln von Parabansäureesterderivat in Parabansäurederivat durch Hydrolyse
iv. Umwandeln von Parabansäurederivat in Parabanacetylchlorid durch Chlorierung
v. Erlangen eines Penicillinderivats auf Parabanbasis aus der Reaktion von Parabanacetylchlorid mit der 6-Aminopenicillinsäure(6-APA)-Verbindung.

2. Verfahren nach Anspruch 1, **dadurch gekennzeichnet, dass** das Harnstoffderivat auf Aminosäurebasis in Vorgangsschritt (i) 2-Phenyl-2-(3-Phenylthioharnstoff)essigsäureester ist und durch die folgende Reaktion synthetisiert wird

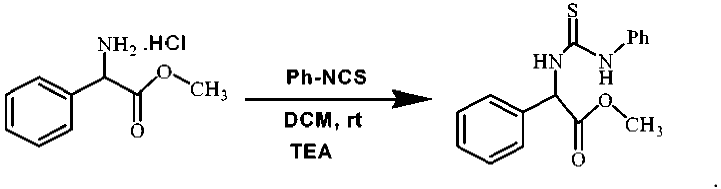

3. Verfahren nach Anspruch 2, **dadurch gekennzeichnet, dass** in Vorgangsschritt Nummer (ii) das Parabansäureesterderivat 2-(4,5-Dioxo-3-phenyl-2-thiooxoimidazolidin-1-yl)-2-(phenyl)essigsäuremethylester ist und durch die folgende Reaktion synthetisiert wird

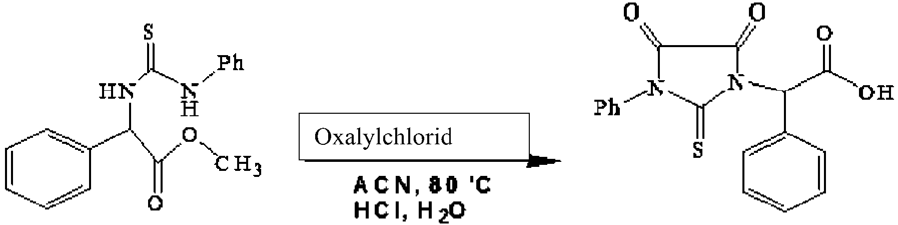

4. Verfahren nach Anspruch 3, **dadurch gekennzeichnet, dass** das Parabansäurederivat in Vorgangsschritt (iii) 2-(4,5-Dioxo-3-phenyl-2-thiooxoimidazolidin-1-yl)-2-(phenyl)essigsäure ist.

5. Verfahren nach Anspruch 4, **dadurch gekennzeichnet, dass in dem** Vorgangsschritt Nummer (iv) der Chlorierungsvorgang mit einer Komponente durchgeführt wird, die aus der Gruppe ausgewählt ist, die aus Thionylchlorid, Phosphorpentachlorid und Phosphortrichlorid besteht.

6. Verfahren nach einem von Anspruch 4 und Anspruch 5, **dadurch gekennzeichnet, dass in dem** Vorgangsschritt Nummer (iv) das Parabanacetylchlorid 2-(4,5-Dioxo-3-phenyl-2-thiooxoimidazolidin-1-yl)-2-(phenyl)acetylchlorid ist und durch die folgende Reaktion synthetisiert wird

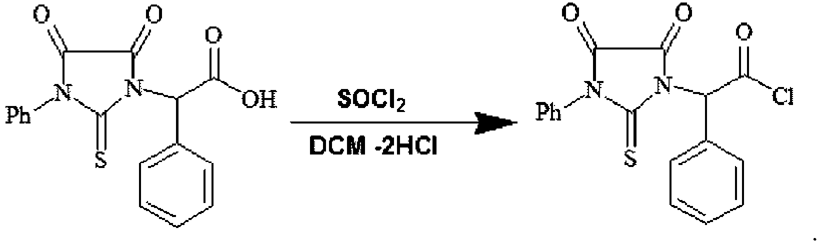

7. Verfahren nach Anspruch 6, **dadurch gekennzeichnet, dass in dem** Vorgangsschritt Nummer (v) das Penicillinderivat auf Parabansäurebasis Natrium-6-(2-(4,5-dioxo-3-phenyl-2-thiooxoimidazolidin-1-yl)-2-phenylacetamido)-3,3-dimethyl-7-oxo-4-thya-1-aza-bicyclo[3.2.0]heptan-2-carboxilat ist und durch die folgende Reaktion synthetisiert wird

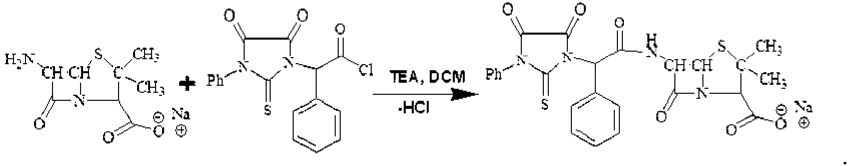

8. Verfahren nach einem der Ansprüche 2 bis 7, **wobei** in den Verbindungen das "S"-Element durch das "O"-Element substituiert wurde.

9. Verfahren zur Synthese von Penicillinderivaten mit der chemischen Formel (II), **dadurch gekennzeichnet, dass es** die folgenden Vorgangsschritte **umfasst;** 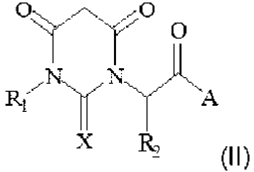 wobei R₁ und R₂ die 4,5,6,7-gliedrigen aromatischen, heterocyclischen oder Alkylgruppen darstellen; A die 6-Aminopenicillinsäure(6-APA)-Verbindung darstellt; X das O- oder S-Element darstellt
i. Erlangen eines Harnstoffderivats auf Aminosäurebasis aus der Reaktion von Aminosäure mit Isocyanat oder Thioisocyanat
ii. Erlangen eines Essigsäureesterderivats auf Pyrimidinbasis aus der Reaktion eines Harnstoffderivats auf Aminosäurebasis mit Malonylchlorid
iii. Umwandeln eines Essigsäureesterderivats auf Pyrimidinbasis in ein Essigsäurederivat auf Pyrimidinbasis durch Hydrolyse
iv. Umwandeln eines Essigsäureesterderivats auf Pyrimidinbasis in Acetylchlorid auf Pyrimidinbasis durch Chlorierung
v. Erlangen eines Penicillinderivats auf Pyrimidinbasis aus der Reaktion von Acetylchlorid auf Pyrimidinbasis mit der 6-Aminopenicillinsäure(6-APA)-Verbindung.

10. Verfahren nach Anspruch 9, **dadurch gekennzeichnet, dass** das Harnstoffderivat auf Aminosäurebasis in Vorgangsschritt (i) 2-Phenyl-2-(3-phenylthioharnstoff)essigsäureester ist und durch die folgende Reaktion synthetisiert wird

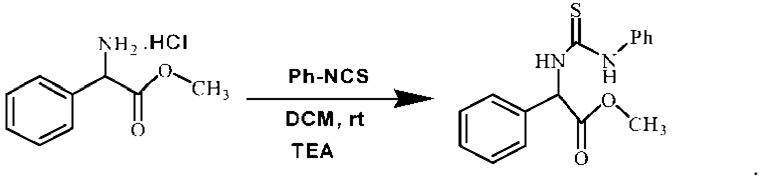

11. Verfahren nach Anspruch 10, **dadurch gekennzeichnet, dass** in Vorgangsschritt Nummer (ii) das Essigsäureesterderivat auf Pyrimidinbasis 2-(4,6-Dioxo-3-phenyl-2-thioxo-tetrahidropyrimidin-1-(2H)-yl)-2-phenylessigsäuremethylester ist und durch die folgende Reaktion synthetisiert wird

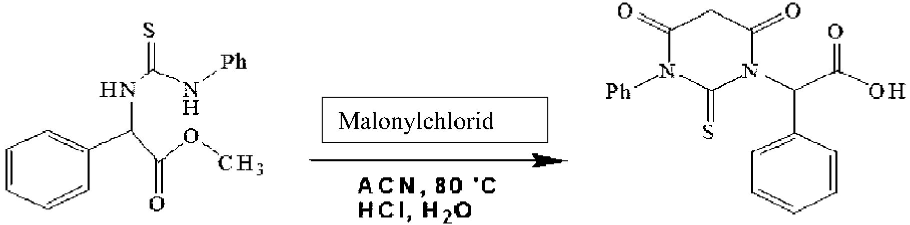

12. Verfahren nach Anspruch 11, **dadurch gekennzeichnet, dass** in Vorgangsschritt (iii) das Essigsäurederivat auf Pyrimidinbasis 2-(4,6-Dioxo-3-phenyl-2-thioxo-tetrahidropyrimidin-1-(2H)-yl)-2-phenylessigsäure ist.

13. Verfahren nach Anspruch 12, **dadurch gekennzeichnet, dass** in Vorgangsschritt Nummer (iv) der Chlorierungsvorgang mit einer Komponente durchgeführt wird, die aus der Gruppe ausgewählt ist, die aus Thionylchlorid, Phosphorpentachlorid und Phosphortrichlorid besteht.

14. Verfahren nach einem von Anspruch 12 bis Anspruch 13, **dadurch gekennzeichnet, dass** in Vorgangsschritt Nummer (iv) das Acetylchlorid auf Pyrimidinbasis 2-(4,6-Dioxo-3-phenyl-2-thioxo-tetrahidropyrimidin-1-(2H)-yl)-2-phenylacetyl ist und durch die folgende Reaktion synthetisiert wird

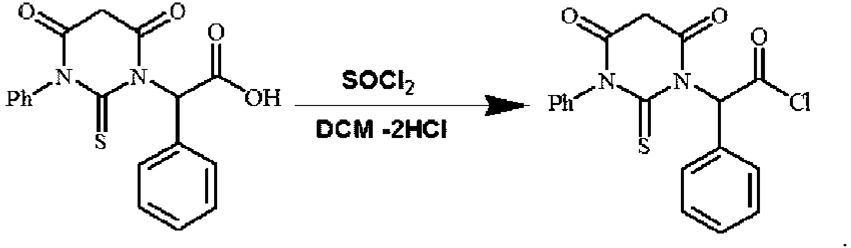

15. Verfahren nach Anspruch 14, **dadurch gekennzeichnet, dass** in Vorgangsschritt (v) das Penicillinderivat auf Pyrimidinbasis Natrium-6-(2-(4,6-dioxo-3-phenyl-2-thioxo-tetrahidropyrimidin-1-(2H)-yl)-2-phenylacetamido)-3,3-dimethyl-7-oxo-4-thya-1-aza-bicyclo[3.2.0]heptan-2-carboxilat ist und durch die folgende Reaktion synthetisiert wird

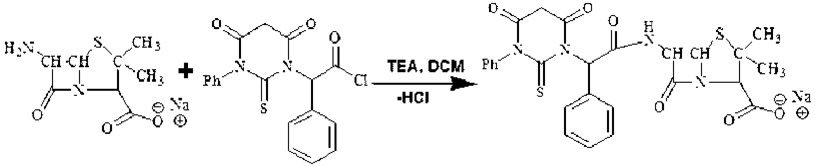

16. Verfahren nach einem von Anspruch 10 bis Anspruch 15, **wobei** in den Verbindungen das "S'"-Element durch das "O"-Element substituiert wurde.

## Revendications

1. Procédé permettant la synthèse de dérivés de pénicilline présentant la formule chimique (I) **caractérisé en ce qu'il comprend** les étapes de processus de ; 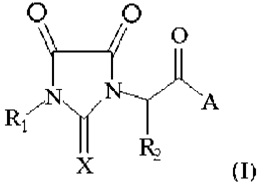 dans lequel R₁ et R₂ représentent les groupes aromatiques, hétérocycliques ou alkyles à 4, 5, 6, 7 chaînons ; A représente le composé acide 6-aminopénicillinique (6-APA) ; X représente l'élément O ou S
i. obtention d'un dérivé d'urée à base d'acides aminés à partir de la réaction d'un acide aminé avec un isocyanate ou un thioisocyanate
ii. obtention d'un dérivé d'ester d'acide parabanique à partir de la réaction de l'urée à base d'acides aminés avec le chlorure d'oxalyle
iii. conversion d'un dérivé d'ester d'acide parabanique en dérivé d'acide parabanique par hydrolyse
iv. conversion d'un dérivé d'acide parabanique en chlorure d'acétyle parabanique par chloration
v. obtention d'un dérivé de pénicilline à base parabanique à partir de la réaction du chlorure d'acétyle parabanique avec le composé acide 6-aminopénicillinique (6-APA).

2. Procédé selon la revendication 1, **caractérisé en ce que** dans l'étape du processus numéro (i), ledit dérivé d'urée à base d'acide aminé est l'ester d'acide acétique 2-phényl-2-(3-phénylthiourée) et est synthétisé par la réaction ci-dessous

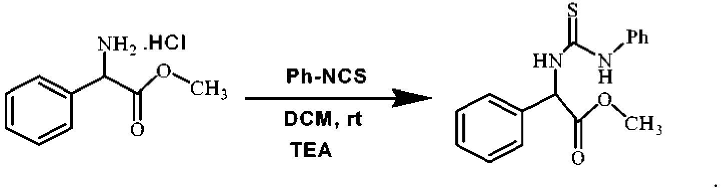

3. Procédé selon la revendication 2, **caractérisé en ce que** dans l'étape du processus numéro (ii), ledit dérivé d'ester d'acide parabanique est l'ester méthylique d'acide acétique 2-(4,5-dioxo-3-phényl-2-thiooxoimidazolidine-1-yl)-2-(phényl) et est synthétisé par la réaction ci-dessous

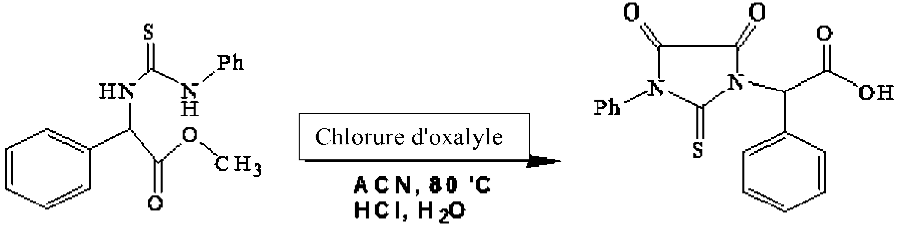

4. Procédé selon la revendication 3, **caractérisé en ce que** dans l'étape du processus numéro (iii), ledit dérivé d'acide parabanique est l'acide acétique 2-(4,5-dioxo-3-phényl-2-thiooxoimidazolidine-1-yl)-2-(phényl).

5. Procédé selon la revendication 4, **caractérisé en ce que** dans l'étape du processus numéro (iv), ledit procédé de chloration est effectué avec un composant choisi dans le groupe constitué du chlorure de thionyle, du pentachlorure de phosphore et du trichlorure de phosphore.

6. Procédé selon l'une quelconque de la revendication 4 et de la revendication 5, **caractérisé en ce que** dans l'étape du processus numéro (iv), ledit chlorure d'acétyle parabanique est le chlorure d'acétyle 2-(4,5-dioxo-3-phényl-2-thiooxoimidazolidine-1-yl)-2-(phényl) et est synthétisé par la réaction ci-dessous

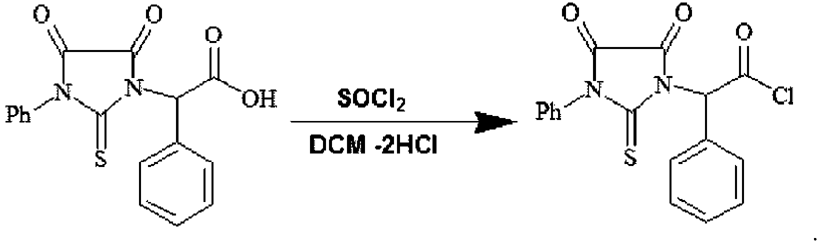

7. Procédé selon la revendication 6, **caractérisé en ce que** dans l'étape du processus numéro (v), ledit dérivé de pénicilline à base d'acide parabanique est le 6-(2-(4,5-dioxo-3-phényl-2-thiooxoimidazolidine-1-yl)-2-phénylacétamido)-3,3-diméthyl-7-oxo-4-thya-1-aza-bicycle [3.2.0] heptane-2-carboxilate de sodium et est synthétisé par la réaction ci-dessous

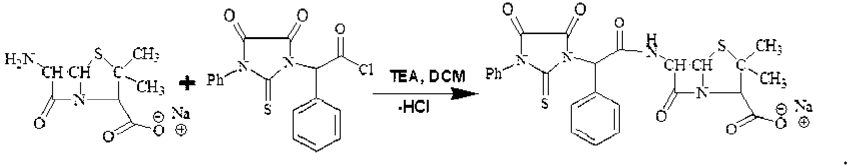

8. Procédé selon l'une quelconque des revendications 2 à 7, **dans lequel** dans les composés ledit élément « S » a été substitué par l'élément « O ».

9. Procédé permettant la synthèse de dérivés de pénicilline présentant la formule chimique (II) **caractérisé en ce qu'il comprend** les étapes de processus de ; 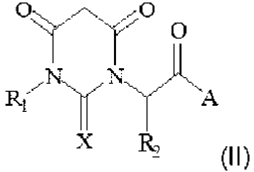 dans lequel R₁ et R₂ représentent les groupes aromatiques, hétérocycliques ou alkyles à 4, 5, 6, 7 chaînons ; A représente le composé acide 6-aminopénicillinique (6-APA) ; X représente l'élément O ou S
i. obtention d'un dérivé d'urée à base d'acides aminés à partir de la réaction d'un acide aminé avec un isocyanate ou un thioisocyanate
ii. obtention d'un dérivé d'ester d'acide acétique à base de pyrimidine à partir de la réaction d'un dérivé d'urée à base d'acides aminés avec le chlorure de malonyle
iii. conversion d'un dérivé d'ester d'acide acétique à base de pyrimidine en dérivé d'acide acétique à base de pyrimidine par hydrolyse
iv. conversion d'un dérivé d'ester d'acide acétique à base de pyrimidine en chlorure d'acétyle à base de pyrimidine par chloration
v. obtention d'un dérivé de pénicilline à base de pyrimidine à partir de la réaction du chlorure d'acétyle à base de pyrimidine avec le composé acide 6-aminopénicillinique (6-APA).

10. Procédé selon la revendication 9, **caractérisé en ce que** dans l'étape du processus numéro (i), ledit dérivé d'urée à base d'acide aminé est l'ester d'acide acétique 2-phényl-2-(3-phénylthiourée) et est synthétisé par la réaction ci-dessous

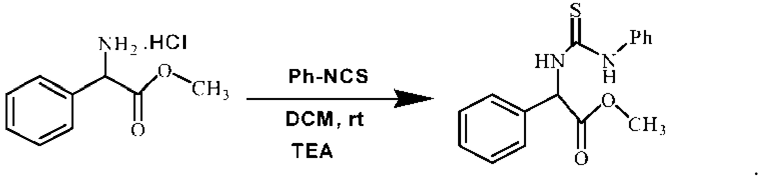

11. Procédé selon la revendication 10, **caractérisé en ce que** dans l'étape du processus numéro (ii), ledit dérivé d'ester d'acide acétique à base de pyrimidine est l'ester méthylique d'acide 2-(4,6-dioxo-3-phényl-2-thioxo-tétrahidropyrimidine-1-(2H)-yl)-2-phénylacétique et est synthétisé par la réaction ci-dessous

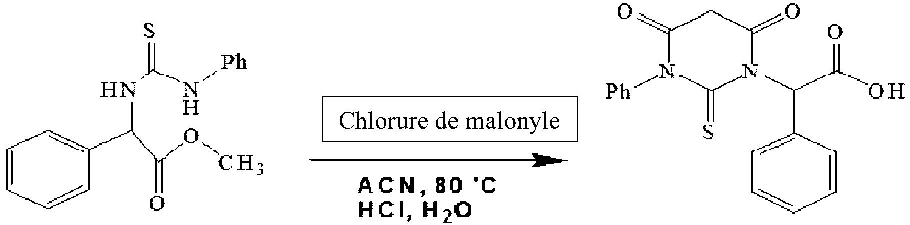

12. Procédé selon la revendication 11, **caractérisé en ce que** dans l'étape du processus numéro (iii), ledit dérivé d'acide acétique à base de pyrimidine est l'acide 2-(4,6-dioxo-3-phényl-2-thioxo-tétrahidropyrimidine-1-(2H)-yl)-2-phénylacétique.

13. Procédé selon la revendication 12, **caractérisé en ce que** dans l'étape du processus numéro (iv), ledit procédé de chloration est effectué avec un composant choisi dans le groupe constitué du chlorure de thionyle, du pentachlorure de phosphore et du trichlorure de phosphore.

14. Procédé selon l'une quelconque de la revendication 12 à la revendication 13 **caractérisé en ce que** dans l'étape du processus numéro (iv), ledit chlorure d'acétyle à base de pyrimidine est le 2-(4,6-dioxo-3-phényl-2-thioxo-tétrahidropyrimidine-1-(2H)-yl)-2-phénylacétyle et est synthétisé par la réaction ci-dessous

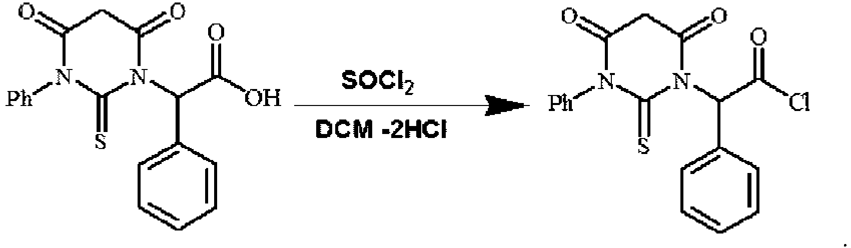

15. Procédé selon la revendication 14, **caractérisé en ce que** dans l'étape du processus numéro (v), ledit dérivé de pénicilline à base de pyrimidine est le 6-(2-(4,6-dioxo-3-phényl-2-thioxo-tétrahidropyrimidine-1-(2H)-yl)-2-phénylacétamido)-3,3-diméthyl-7-oxo-4-thya-1-aza-bicycle[3.2.0]heptane-2-carboxilate de sodium et est synthétisé par la réaction ci-dessous

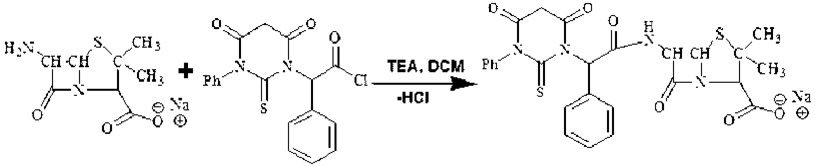

16. Procédé selon l'une quelconque des revendications 10 à 15, **dans lequel** dans les composés ledit élément « S » a été substitué par l'élément « O ».
